# EUROPEAN PATENT APPLICATION

(11) **EP 3 804 630 A1**
(43) Date of publication of application: **14.04.2021**
(21) Application number: 19202477.6
(22) Date of filing: 10.10.2019
(51) Int. Cl.: A61B 8/08, A61B 8/00, A61B 90/00

(54) **ULTRASOUND OBJECT ZOOM TRACKING**

(71) Applicant: Koninklijke Philips N.V., 5656 AG Eindhoven (NL)
(72) Inventor: POLAND, McKee Dunn, 5656 AE Eindhoven (NL); BELLAMINE, Wadii, 5656 AE Eindhoven (NL)
(74) Representative: Philips Intellectual Property & Standards

(57) **Abstract**

An ultrasound device is provided for imaging an anatomical region. The ultrasound device includes an ultrasound probe (288) having a medical instrument (202) in communication therewith, and an ultrasound sensor (279) mounted on the medical instrument. The ultrasound device further includes a hardware processor (214) configured to render a Region Of Interest (ROI) relative to a tip of the medical instrument on an ultrasound image displayed on a display device, and automatically and selectively perform a ROI refinement by positioning a displayed portion of the scanned image such that the ROI is located in a preferred specific region of the displayed portion of the scanned image. The displayed portion of the scanned image is a selectively magnifiable area within an area of the anatomical region including the medical instrument and spanned by the ultrasound scan line pattern.

## Description

### BACKGROUND OF THE INVENTION

### Technical Field

This disclosure relates to object detection and more particularly to ultrasound object zoom tracking.

### Description of the Related Art

A primary problem in ultrasound object tracking systems occurs when the tracked object, e.g., the tip of the needle, moves across the image as the object is advanced in the tissue, e.g. as the needle is pushed into the tissue. In current systems (regardless of whether the object is tracked), when video zoom is active (e.g. enabled via a gesture using a User Interface (UI)), and especially if the magnification factor is high, then small movements of the needle or probe, whether intentional or not, may move the needle tip out of the magnified image area displayed.

As such, there is a need for an ultrasound object zoom tracking that addresses these deficiencies.

### SUMMARY OF THE INVENTION

According to an aspect of the present invention, an ultrasound device is provided for imaging an anatomical region. The ultrasound device includes an ultrasound probe having a medical instrument in communication therewith, and an ultrasound sensor mounted on the medical instrument. The ultrasound device further includes a hardware processor configured to render a Region Of Interest (ROI) relative to a tip of the medical instrument on an ultrasound image displayed on a display device, and automatically and selectively perform a ROI refinement by positioning a displayed portion of the scanned image such that the ROI is located in a preferred specific region of the displayed portion of the scanned image. The displayed portion of the scanned image is a selectively magnifiable area within an area of the anatomical region including the medical instrument and spanned by the ultrasound scan line pattern.

According to another aspect of the present invention, an ultrasound system is provided for imaging an anatomical region. The ultrasound system includes an ultrasound probe having a medical instrument in communication therewith, and an ultrasound sensor mounted on the medical instrument. The ultrasound system further includes a display configured to display an ultrasound scan line pattern, that spans an area of the anatomical region including the medical instrument, as a scanned image showing a position of the medical instrument by rendering an ROI on the scanned image. The ultrasound system also includes a hardware processor configured to automatically and selectively perform a ROI refinement by positioning a displayed portion of the scanned image such that the ROI is located in a preferred specific region of the displayed portion of the scanned image. The displayed portion of the scanned image is a selectively magnifiable area within an area of the anatomical region including the medical instrument and spanned by the ultrasound scan line pattern.

According to yet another aspect of the present invention, in an ultrasound system that (a) tracks a medical instrument in an anatomical region using (i) an ultrasound sensor mounted on the medical instrument and (ii) an ultrasound scan line pattern that spans an area of the anatomical region including the medical instrument, and (b) displays the ultrasound scan line pattern as a scanned image showing a position of the medical instrument by rendering an ROI on the scanned image, a method is provided. The method includes automatically and selectively performing a ROI refinement by positioning a displayed portion of the scanned image such that the ROI is located in a preferred specific region of the displayed portion of the scanned image. The displayed portion of the scanned image is a selectively magnifiable area within the area of the anatomical region including the medical instrument and spanned by the ultrasound scan line pattern.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
Fig. 1 is a block diagram showing an exemplary processing system, in accordance with an embodiment of the present invention;
Fig. 2 is a block diagram showing an acoustic shape sensing system which employs a tip configuration, in accordance with an embodiment of the present invention;
Figs. 3-5 are flow diagrams showing an exemplary method for ultrasound object zoom tracking, in accordance with an embodiment of the present invention;
Fig. 6 is a diagram showing an object and Region Of Interest (ROI) corresponding to a non-refinement stage, in accordance with an embodiment of the present invention;
Fig. 7 is a diagram showing the object of FIG. 6 and a dynamically refined Region Of Interest (ROI) corresponding to a refinement stage, in accordance with an embodiment of the present invention;
Fig. 8 is a diagram showing the object of FIG. 6 and a dynamically refined Region Of Interest (ROI) corresponding to a refinement stage, in accordance with an embodiment of the present invention; and
Fig. 9 is a block diagram showing an apparatus for ultrasound object zoom tracking, in accordance with an embodiment of the present invention.

### DETAILED DESCRIPTION OF EMBODIMENTS

Embodiments of the present invention are directed to ultrasound object zoom tracking.

Advantageously, embodiments of the present invention can keep the tracked location of the object in the field of view, especially when the image is zoomed such that there is the option of panning. Further, the present invention can place a panned image such that the tracked object is displaced from the image center so that central part of the image shows the tissue that will be traversed when the object is physically advanced in the tissue. These and other aspects of the present invention are described herein.

In an embodiment, the present invention automatically moves the field of view in coordination with the position of the tracked object. The invention is particularly applicable when the system has magnified the image via a video zoom function, meaning that scanned image data in both lateral and depth dimensions is available but off-screen. The automatic panning and zoom functions of the invention take advantage of the scanned data by moving it in and out of the displayed image area. These and other advantages and features of the present invention are described herein.

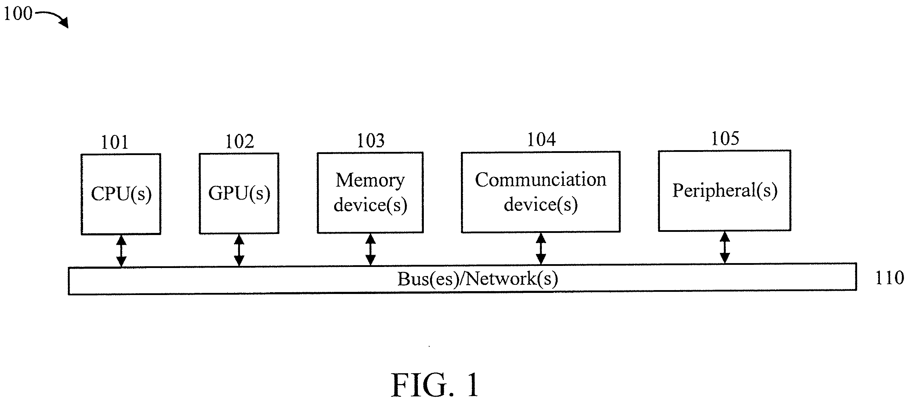

Fig. 1 is a block diagram showing an exemplary processing system 100 to which aspects of the present invention may be applied. The processing system 100 includes a set of processing units (e.g., CPUs) 101, a set of GPUs 102, a set of memory devices 103, a set of communication devices 104, and set of peripherals 105. The CPUs 101 can be single or multi-core CPUs. The GPUs 102 can be single or multi-core GPUs. The one or more memory devices 103 can include caches, RAMs, ROMs, and other memories (flash, optical, magnetic, etc.). The communication devices 104 can include wireless and/or wired communication devices (e.g., network (e.g., WIFI, etc.) adapters, etc.). The peripherals 105 can include a display device, a user input device, a printer, an imaging device, and so forth. Elements of processing system 100 are connected by one or more buses or networks (collectively denoted by the figure reference numeral 110).

In an embodiment, memory devices 103 can store specially programmed software modules to transform the computer processing system into a special purpose computer configured to implement various aspects of the present invention. In an embodiment, special purpose hardware (e.g., Application Specific Integrated Circuits, and so forth) can be used to implement various aspects of the present invention.

Of course, the processing system 100 may also include other elements (not shown), as well as omit certain elements. For example, various other input devices and/or output devices can be included in processing system 100, depending upon the particular implementation of the same. For example, various types of wireless and/or wired input and/or output devices can be used. Moreover, additional processors, controllers, memories, and so forth, in various configurations can also be utilized. These and other variations of the processing system 100 are contemplated given the present teachings.

Moreover, it is to be appreciated that various figures as described below with respect to various elements and steps relating to aspects of the present invention that may be implemented, in whole or in part, by one or more of the elements of system 100.

Computer processing system 100 can be part of an ultrasound system implementing one or more aspects of the present invention.

A further description will now be given regarding various aspects of the present invention.

Embodiments of the present invention can both magnify and/or pan a ROI shape. Embodiments of the present invention can pan and zoom the ultrasound image showing the tracked object in accordance with the position of the object on the image display. In an embodiment, the object is a needle tip.

For the purpose of illustration, the present invention will be described in the context of an embodiment involving Needle Tip Tracking (NTT). The zoom feature is active, and it automatically pans the 2D image to keep the NTT shape (e.g., a circle, a triangle, a square, etc.) near the middle of the displayed image. The zoom feature can also control the zoom factor itself. Thus, the present invention will automatically follow the location of the needle tip, keeping the magnified image around the needle tip in view. Additionally, because the system performs a linear regression on the series of needle location points detected, and determines the trajectory slope, e.g., the functionality to determine the trajectory that the needle will follow as it is pushed, the present invention may also pan the image so as to displace the needle tip location near one corner of the displayed image instead of center, allowing the clinician to visualize more of the magnified tissue that the needle will move into when further inserted.

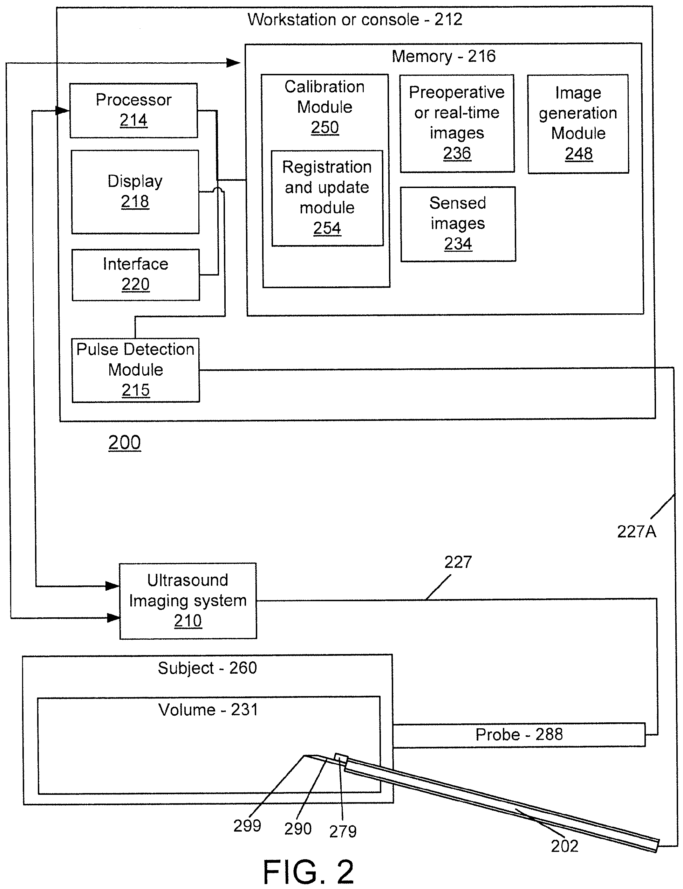

Fig. 2 is a block diagram showing an ultrasound system 200 which employs a tip configuration, in accordance with an embodiment of the present invention.

System 200 may include a workstation or console (hereinafter "workstation") 212 from which a procedure is supervised and/or managed. In an embodiment, one or more elements of system 200 can be implemented by computer processing system 100 of Fig. 1. Workstation 212 preferably includes one or more processors 214 and memory 216 for storing programs and applications. In particular, the memory 216 includes a calibration module 250 (in turn, including a registration and update module 254), pre-operative or real time images 236, an image generation module 248, and sensed images 234.

System 200 includes other elements such as a display 218, an interface 220, a pulse detection module 215, an ultrasound imaging system 210, an ultrasound probe 288, and a medical or interventional device (hereinafter medical device) 202. Various elements of system 200, such as, but not limited to, pulse detection module 215, can have its own memory and controller, depending upon the implementation.

The pulse detection module 215 interprets ultrasound feedback signals from ultrasound probe 288 and an ultrasound sensor 279 mounted on medical device 202.

Pulse detection module 215 can use ultrasound signal feedback (and/or any other feedback) to reconstruct changes in position associated with a medical device 202 in its surrounding region (e.g., volume 231) relative to the ultrasound probe 288, by means of analyzing signals from sensor 279 mounted on the medical device 202 as described further below.

The medical device 202 may include a catheter, a guidewire, an endoscope, a robot, an electrode, a filter device, a balloon device, a needle, or other medical component, etc. The preceding devices are merely illustrative and, thus, other devices can also be used with medical device 202. For the sake of illustration, medical device 202 includes a needle having a tip 299. The ultrasound sensor 279 is mounted on or proximate to the needle. Another medical device 202 such as, for example, a catheter, would also have the ultrasound sensor 279 mounted on the catheter. The ultrasound sensor 279 could be mounted on different positions of the medical device 202, depending upon the particular implementation.

The ultrasound probe 288 generates ultrasound signals which are reflected by structures in a patient's body in order to reproduce the structures in image form on ultrasound image system 210.

The ultrasound sensor 279 preferably operates in a receive only mode. In particular, the ultrasound sensor 279 receives acoustic pulse signals from ultrasound probe 288 as it performs ultrasound transmit pulse generation and received echo beamforming. The resulting acoustic transmit-receive scan lines provide the acoustic signals utilized by the ultrasound imaging system 210 to generate image data to be rendered and displayed on the ultrasound imaging system 210, the workstation 212, or both. The ultrasound sensor 279 likewise receives acoustic pulses from the scan lines generated by probe 288, and sends the resulting receive signals to pulse detection module 215, where it thereby calculates the position of the ultrasound sensor 279, and thus of medical device 202 (and in this example, its needle tip 299), with respect to ultrasound probe 288, and thus with respect to the image of the volume 231 that is being imaged by ultrasound probe 288. Furthermore, the pulse detection module 215 may reconstruct a path of position changes of ultrasound sensor 279, and thus of medical device 202 (and in this example, its needle tip 299), as mentioned previously.

The scan line pattern generated by probe 288 in conjunction with imaging system 210 may encompass a 2D planar extent or a 3D volume extent. For simplicity of explanation, the description of the present invention will assume a 2D scan line pattern and thus use the terms "depth" and "lateral extent" to describe the locus of scan lines in the scan line pattern. However, the same principles of the present invention may equally apply for scan line patterns that are 3D, hence with depth extent, lateral extent, and elevation extent, for example. That is, zoom and pan actions of the present invention may be calculated in 3D and used to magnify and shift portions of a 3D rendered ultrasound image.

Ultrasound probe 288 is connected to the workstation 212 through cabling 227, via the ultrasound imaging system 210. Medical device 202 is connected to pulse detection module 215 through cabling 227A. The cabling 227 and/or 227A may include fiber optics, electrical connections, other instrumentation, and so forth, as needed.

In one embodiment, workstation 212 includes an image generation module 248 configured to receive feedback from the ultrasound probe 288 and record accumulated position data as to where the medical device 202 has been within the volume 231. The ultrasound imaging system 210 is a source of images 236 upon which the overlap of the needle (or whatever tracked medical device 202) tip location ROI is placed. A series of sensed images 234 of a history 236 of the ultrasound probe 288 within the space or volume 231 can also be displayed on a display device 218. Workstation 212 includes the display device 218 for viewing internal images of a subject (patient) or volume 231 and may include the sensed images 234 as an overlay or other rendering of the history 236 of visited positions of the medical device 202 in addition to the current displayed position of the device. Display 218 may also permit a user to interact with the workstation 212 and its components and functions, or any other element within the system 200. This is further facilitated by an interface 220 which may include a keyboard, mouse, a joystick, a haptic device, or any other peripheral or control to permit user feedback from and interaction with the workstation 212.

The ultrasound probe 288 is connected to the ultrasound imaging system 210 which among other things acts a source of ultrasonic pulses (e.g., a pulse generator) in conjunction with ultrasound probe 288. The imaging system and ultrasound probe 288 work together to generate acoustic scan lines, including transmit and receive phases, typically including phased array beamforming techniques to form beams which acoustically interrogate volume 231 (or a locus of scan lines therein) to collect echo data for the formation of images 236 as a series of acoustic frames. In the process of transmitting acoustic pulses for scan lines, probe 288 insonicates the region in volume 231 where medical device 202 is located, and thus also insonicates ultrasound sensor 279. Signals from sensor 279 are sent to pulse detection module 215 which, in conjunction with timing information from ultrasound imaging system 210, may determine the location of the tip of medical device 202 in the field of view imaged by probe 288.

In an embodiment, a needle is mounted at a distal end of the medical device 202, for example, at the tip 299 thereof. Again, other devices can be used in place of a needle as medical device 202. In such a case, ultrasound sensor 279 may be located at a different location than it would on a needle. The ultrasound sensor 279 mounted on or proximate to the needle 290 or other device detects the location of the tip 299 of the needle or other device and provides tip location information to the workstation 212. The processor 214 may be used to generate a Region of Interest (ROI) circle.

As described above, system 200 measures and analyzes acoustic pulses observed by the needle using components such as pulse detection module 215, and by timing the acoustic pulses with respect to the timing of the scan lines in the ultrasound probe's 288 acoustic frame, and placing the location of the ultrasound sensor both in azimuth and depth on the rendered image. Thus, the position of the ultrasound sensor respective to the ultrasound probe 288 is determined based on ultrasound signals detected by the ultrasound sensor 279 on the needle.

Thus, for the sake of illustration, the medical device 202 is described as a needle. If the clinical application is regional anesthesia, anesthetic is pumped through a cannula of the needle to surround a nerve bundle, and then the needle is withdrawn and a procedure can be done by a qualified medical person. The exact location of the needle tip as seen in the ultrasound image is critical for effectiveness and safety.

On the other hand, if the clinical application is, for example, insertion of a catheter, such as for minimally invasive surgical intervention, then the needle's function is to create the puncture, lead a guide wire into a vessel, get withdrawn, and then the catheter itself is pushed into the vessel using the guide wire. In that case, the catheter that would have the ultrasound sensor 279 mounted on it, instead of (or in addition to) the needle.

These and other medical devices 202 to which the present invention can be applied are readily determined given the teachings relating to various embodiments of the present invention described herein.

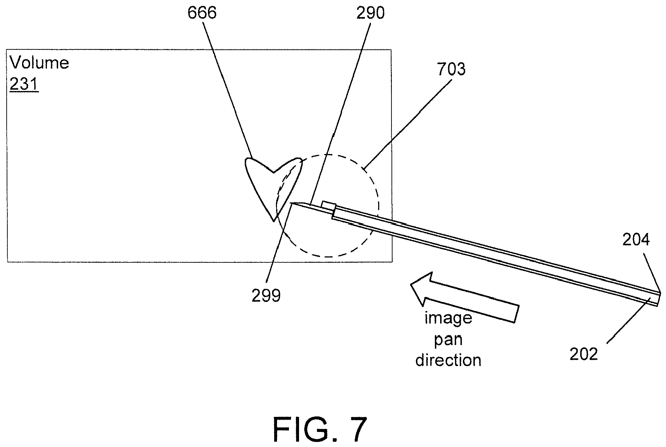

While display 218 and ultrasound imaging device 210 are described herein for functions relating to displaying images, in other embodiments, a single display device can be used. For example, in an embodiment, the display 218 can be omitted, and the ultrasound imaging system 210 can provide ultrasound probe signal generation (pulse transmit), reception (pulse receive), signal demodulation and detection, filtering, and so forth, implementing a standard ultrasound signal path. The resulting detected, filtered signal stream goes into scan conversion, Doppler detection, rendering, and so forth and can be displayed within the same "system". In an embodiment, the ultrasound probe can just be a mechanical housing with some simple circuitry to drive the (typically piezoelectric) transducer elements, e.g., 128 of them. In an embodiment (e.g., system 700 of Fig. 7)), most of the ultrasound signal path (without the display) can be located in the ultrasound probe handle itself to provide a compact system.

These and other variations of system 200 are readily determined given the teachings relating to various embodiments of the present invention described herein.

A further description will now be given regarding various aspects of the present invention.

The present invention changes no aspects of the image processing or acquisition line pattern, just the zoom pan coordinates and optionally also the zoom magnification level, as described further below. The pan behavior can be achieved by mapping the NTT rendered circle position to the pan position stimulus. Thus, it is a quick and practical enhancement to implement.

The present invention builds upon the existing video zoom capability in the ultrasound system. Video zoom itself operates only up to the limits of the scan sweep by the probe, hence the present invention also has that limit. So, as the probe is moved laterally or the needle tip moves out to the edge of the probe's scan line pattern, the pan position will follow until it stops at its maximum offset, according to logic already in the system that limits the user's panning. As an enhancement, one border side of the zoomed display area may be colored to alert the user that the needle tip is approaching the edge of the probe's scan view, prompting the user to slide the probe laterally to follow.

When the needle tip goes out of the scan line plane generated by probe 288 in conjunction with imaging system 210, typically as a result of tilting the probe in elevation, the NTT signal weakens, resulting in a reduced signal to noise ratio (SNR). In an embodiment, the NTT ROI shape (e.g. a circle) progressively widens, changes color, and disappears accordingly with decreasing SNR, under control of pulse detection module 215 and registration and update module 254. To minimize unnecessary panning, the present invention can hold its last pan position when the NTT signal falls below a threshold SNR, updating the pan position only when the NTT position is re-acquired with sufficient SNR. This will prevent the pan position from jittering.

The pan movements may be strongly low-pass filtered to again add stability and smoothness to the automatic panning movement.

Any of a variety of mechanisms implement arbitration between automatic panning by the present invention and manual panning (by gesture or trackball). For example, in an embodiment, manual panning input may just disable the present invention, obliging the user to turn it back on by pressing its icon when automatic panning is again preferred. In an embodiment, a manual panning override may persist until the needle tip position itself moves within the scan line pattern, at which point the present invention takes over again. This last behavior would suit the workflow where the clinician manually pans forward in the needle path to see the next anatomy, magnified, that the needle will reach, but only wants to hold that view until the needle advances, at which point the present invention resumes its action and centers the needle tip in the view. In an embodiment, a button on the user interface could turn the present invention on and off. Of course, other behaviors/mechanisms can also be implemented.

Optionally, the user may select a behavior that zooms in automatically along with the present invention centering action when the NTT position strength rises above a threshold SNR, and zoom back out when strength falls below an SNR threshold. The SNR thresholds could be 80% for rise, 40% for fall, thereby providing hysteresis and preventing the zoom level from toggling between magnifications rapidly. Of course, other percentages can be used. The benefit of this enhancement is that when the needle tip position is successfully tracked, the user immediately sees centered zoom detail of the surrounding tissue, but when the needle tip is lost (out of plane, past the scan line pattern, hidden in an anatomical void), then the system reverts back to 1.0 magnification, to show the landscape anatomy which will help to find the needle again.

The behaviors of the present invention described thus far may be collectively called feature 1. When the present invention is active, an on-screen indicator (of feature 1) could make it obvious, such as a special color of the border of the display area, in addition to an icon.

A variant of feature 1 could be called feature 2. In this feature 2 mode, the ROI position circle (e.g. for the exemplary NTT function, as described above) is always at the center of the display, and the underlying image, regardless of zoom, is panned underneath as the needle tip moves. Again, the extents of the scan line pattern dictate the boundaries of the displayed image. The needle tip may be advanced through tissue to approach a boundary in scan line depth or lateral extent. It will be obvious that the needle tip is nearing the edge of the displayable image as that edge of the image moves inwards on the display towards the circle locked at the center of the screen. Sliding the probe in response would correctly fill in the missing part of the image as the needle tip position returns to the center of the scan line pattern. Feature 2 would work identically at any magnification.

Features of the present invention work in alternative ways to allow the NTT circle to be centered on the display. In one way, the present invention limits the panning offset so that the whole image area on the display is always occupied by available scan lines, and requires some level of zoom magnification to have more active image area to pan into the display. In another way, zoom is not required, instead allowing the boundary of the image to move toward center (axially or laterally as it runs out of depth or extent of scan lines, respectively), which effectively truncates the image area, prompting the user to move the probe in the right direction to see the new territory the needle is entering. To summarize, the NTT shape (e.g., a circle) is preferentially centered on the screen. When zoomed in, the image area will always be fully populated, but when zoomed out to 1.0 magnification, the image area may be truncated if the needle tip is near the edge of the scan line pattern, intuitively prompting the user to move the probe.

Finally, wherever the description herein mentions putting the ROI NTT circle at the center of the image, it could be placed instead near a corner of the image, so that the next tissue reached by advancing the needle occupies the majority of the display area. The corner would be typically upper left or upper right, and the discrimination between those two is determined by the slope of the needle advance trajectory, which is an output of the trajectory determining algorithm.

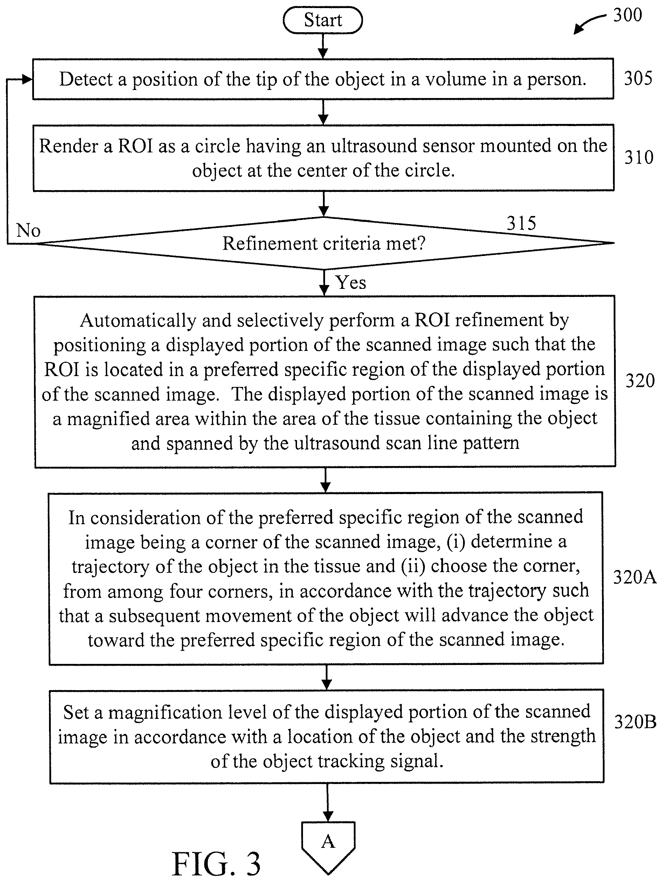

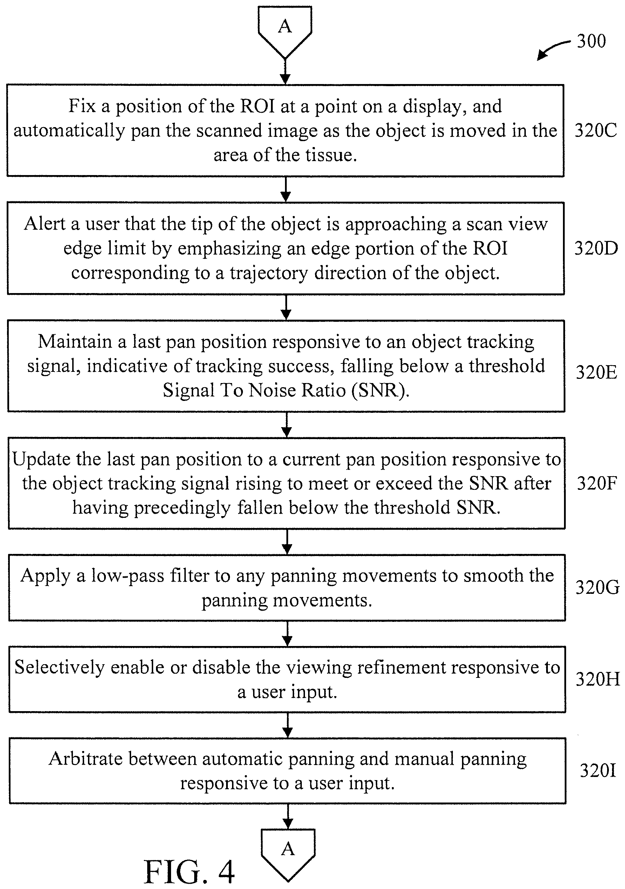

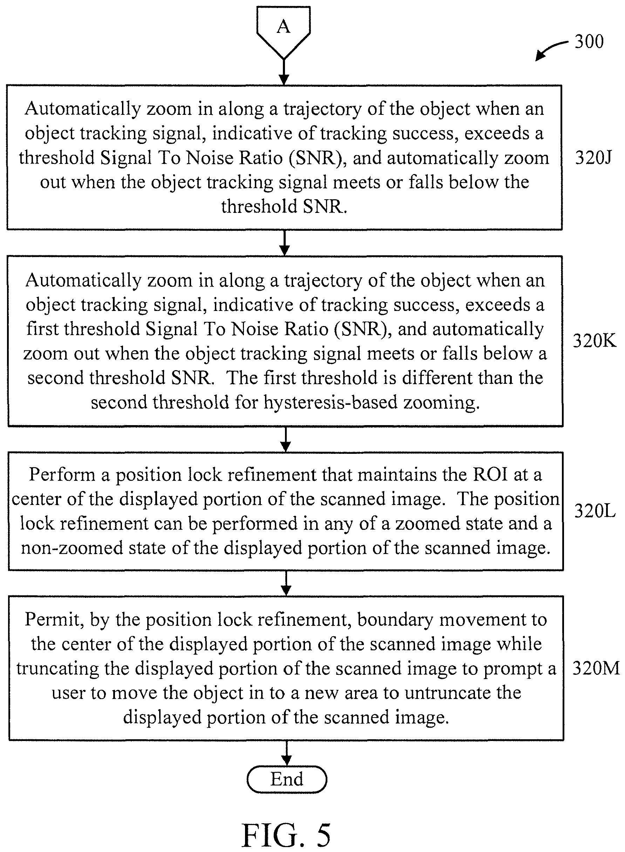

Figs. 3-5 are flow diagrams showing an exemplary method 300 for ultrasound object zoom tracking, in accordance with an embodiment of the present invention. The method 300 is performed in an ultrasound system that tracks an object in a tissue using an ultrasound sensor mounted on a tip of the object and displays a position of the object by rendering a Region Of Interest (ROI) relative to the tip on an ultrasound image displayed on a display device.

At block 305, detect a position of the tip of the object in a volume in a person.

At block 310, render a ROI as a circle having an ultrasound sensor mounted on the object at the center of the circle.

At block 315, determine if refinement criteria have been met. If so, then proceed to block 320. Otherwise, return to block 305.

At block 320, automatically and selectively perform a ROI refinement by positioning a displayed portion of the scanned image such that the ROI is located in a preferred specific region of the displayed portion of the scanned image. The displayed portion of the scanned image is a magnified area within the area of the tissue including the object and spanned by the ultrasound scan line pattern.

In an embodiment, block 320 can include one or more of block 320A-320M.

At block 320A, in consideration of the preferred specific region of the scanned image being a corner of the scanned image, (i) determine a trajectory of the object in the tissue and (ii) choose the corner, from among four corners, in accordance with the trajectory such that a subsequent movement of the object will advance the object toward the preferred specific region of the scanned image. For example, use the chosen corner in step 320C.

At block 320B, set a magnification level of the displayed portion of the scanned image in accordance with a location of the object and the strength of the object tracking signal.

At block 320C, fix a position of the ROI at a point on a display, and automatically pan the scanned image as the object is moved in the area of the tissue.

At block 320D, alert a user that the tip of the object is approaching a scan view edge limit by emphasizing an edge portion of the ROI corresponding to a trajectory direction of the object.

At block 320E, maintain a last pan position responsive to an object tracking signal, indicative of tracking success, falling below a threshold Signal To Noise Ratio (SNR).

At block 320F, update the last pan position to a current pan position responsive to the object tracking signal rising to meet or exceed the SNR after having precedingly fallen below the threshold SNR.

At block 320G, apply a low-pass filter to any panning movements to smooth the panning movements.

At block 320H, selectively enable or disable the viewing refinement responsive to a user input.

At block 3201, arbitrate between automatic panning and manual panning responsive to a user input.

At block 320J, automatically zoom in along a trajectory of the object when an object tracking signal, indicative of tracking success, exceeds a threshold Signal To Noise Ratio (SNR), and automatically zoom out when the object tracking signal meets or falls below the threshold SNR.

At block 320K, automatically zoom in along a trajectory of the object when an object tracking signal, indicative of tracking success, exceeds a first threshold Signal To Noise Ratio (SNR), and automatically zoom out when the object tracking signal meets or falls below a second threshold SNR, the first threshold being different than the second threshold for hysteresis-based zooming.

At block 320L, perform a position lock refinement that maintains the ROI at a center of the displayed portion of the scanned image. The position lock refinement can be performed in any of a zoomed state and a non-zoomed state of the displayed portion of the scanned image.

At block 320M, permit, by the position lock refinement, boundary movement to the center of the displayed portion of the scanned image while truncating the displayed portion of the scanned image to prompt a user to move the object in to a new area to untruncate the displayed portion of the scanned image.

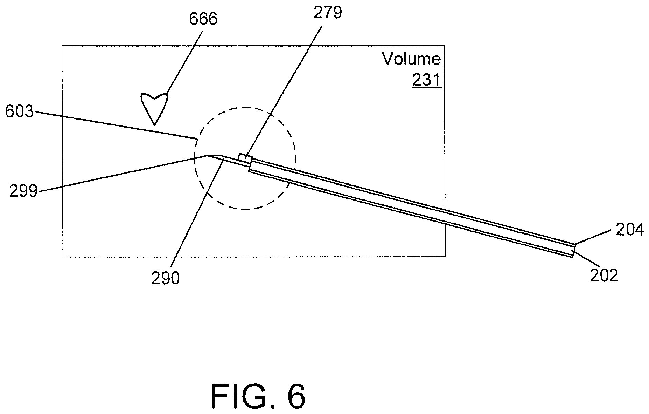

Figs. 6 and 7 respectively show an object and a corresponding ROI circle, corresponding to a non-refinement stage and a refinement stage applied to the ROI circle, in accordance with one or more embodiments of the present invention. It is to be appreciated that the elements in Figs. 6 and 7 may not be drawn to scale, but are enumerated to show the relevant parameters.

Fig. 6 is a diagram showing an object (e.g., a needle) 290 and Region Of Interest (ROI) 603 corresponding to a non-refinement stage, in accordance with an embodiment of the present invention. In an embodiment, FIG. 6 can be considered to show an implementation of block 310 of method 300 of Fig. 3.

In an ultrasound system that tracks an object 290 of an ultrasound sensor 279 mounted near the tip 299 of the object 290, the location of the tip 299 of the object 290 is shown on the image of a small ROI 603 that tracks the tip movement as the object 290 is advanced in the tissue. While the shown ROI is a circle, other shapes can be used. The center of the circle 603 is displayed by the system tracking algorithm at the physical location of the ultrasound sensor 279.

Hence, corresponding to a non-refinement stage of the present invention, ROI circle 603 encompasses the tip 299. As shown, the needle tip 299 is at the perimeter of the ROI circle 603, with the ROI circle 603 being centered on the ultrasound sensor 279 on the needle 290. A human heart 666 is shown unmagnified.

Fig. 7 is a diagram showing the object (e.g., needle 290) of Fig. 6 and a dynamically refined Region Of Interest (ROI)/circle 703 corresponding to a refinement stage, in accordance with an embodiment of the present invention. In an embodiment, Fig. 7 can be considered to show an implementation of block 320 of method 300 of Fig. 3.

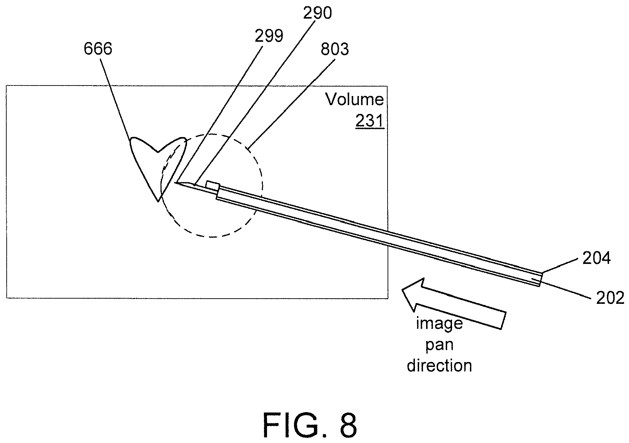

In the refinement stage, a region is zoomed (magnified) and the ROI is located in a preferred specific region of the image. In the embodiment of Fig. 7, the preferred specific region is a corner (i.e., the bottom right corner). In another embodiment, the preferred specific region can be the center of the image (see, e.g., Fig. 8) or some other location. Zooming and panning are performed to magnify the view while maintaining the ROI in the preferred specific region of the image left top corner. The human heart 666 is shown magnified with the tracked object (needle 290) at the preferred specific region of the bottom right corner. Panning is performed from right to left, in the direction of the trajectory of the object. This approach allows the clinician to visualize target tissue that the object will approach.

Fig. 8 is a diagram showing the object (e.g., needle 290) of Fig. 6 and a dynamically refined Region Of Interest (ROI)/circle 803 corresponding to a refinement stage, in accordance with an embodiment of the present invention. In an embodiment, Fig. 8 can be considered to show an implementation of block 320 of method 300 of Fig. 3.

In the refinement stage, a region is zoomed (magnified) and the ROI is located in a preferred specific region of the image. In the embodiment of Fig. 8, the preferred specific region is a center of the displayed area. Zooming and panning are performed in order to magnify the view while maintaining the ROI in the preferred specific region of the image center. The human heart 666 is shown magnified with the tracked object (needle 290) at the preferred specific region of the image center. Panning is performed from right to left, in the direction of the trajectory of the object. This approach allows the clinician to visualize target tissue that the object will approach.

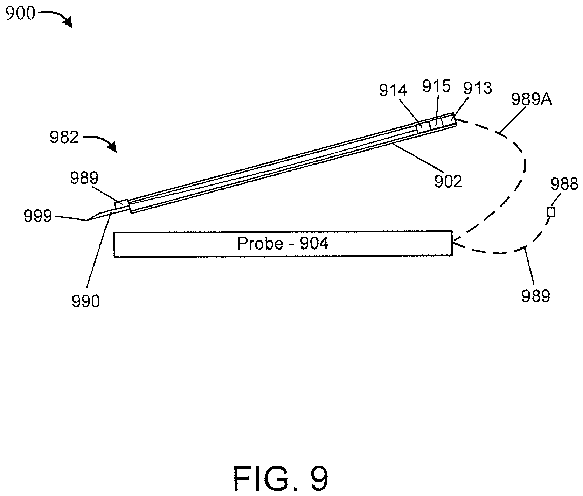

Fig. 9 is a block diagram showing an apparatus 900 for ultrasound object zoom tracking, in accordance with an embodiment of the present invention.

The system 900 includes an ultrasound probe 904 that cooperates with a medical instrument 902. The medical instrument 902 includes an ultrasound sensor 989 mounted on, e.g., a tip 999 of, the medical instrument 902.

The system 900 further includes a hardware processor 914, a memory 913, and a pulse detection module 915 which cooperate to render a Region Of Interest (ROI) relative to the tip on an ultrasound image displayed on a display device, and selectively perform a dynamic ROI refinement as the medical instrument is moved through the tissue. The hardware processor 914, memory 913, and pulse detection module 915 may be part of a circuit subsystem 982. Circuit subsystem 982 can include one or more elements of Fig. 2, depending upon the implementation, in order to obtain a compact system 900 relative to system 200. For example, while system 200 is envisioned in one embodiment being implementing using a cart specifically configured for use with system 200, in other embodiments, the ultrasound probe 904 can simply be connected to any display device. To that end, it is envisioned that circuit subsystem 982 at least includes ultrasound sensing module 915.

The medical instrument 902 may further include a catheter, a guidewire, a probe, an endoscope, a robot, an electrode, a filter device, a balloon device, or other medical component, etc. For the sake of illustration, medical instrument 902 includes a needle 990 having a tip 999. The preceding instruments are merely illustrative and, thus, other instruments can also be used with medical instrument 902.

Cabling 989 and connector (e.g., USB, etc.) 988 can be used to connect ultrasonic probe 904 to a display device and/or other elements. Cabling 989A can be used to connect ultrasonic probe 904 to medical instrument 902, in particular, pulse detection module 915. In other embodiments, wireless communication can be used.

In one example of the disclosure an ultrasound system for imaging an anatomical region, comprises: an ultrasound probe (204) having a medical instrument (202) in communication therewith, and an ultrasound sensor (279) mounted on the medical instrument; a display (218) configured to display an ultrasound scan line pattern, that spans an area of the anatomical region including the medical instrument, as a scanned image showing a position of the medical instrument by rendering an ROI on the scanned image; and a hardware processor (214) configured to automatically and selectively perform a ROI refinement by positioning a displayed portion of the scanned image such that the ROI is located in a preferred specific region of the displayed portion of the scanned image, wherein the displayed portion of the scanned image is a selectively magnifiable area within an area of the anatomical region including the medical instrument and spanned by the ultrasound scan line pattern. In this example the hardware processor (214) sets a magnification level of the displayed portion of the scanned image in accordance with a location of the medical instrument.

In another example of the disclosure, in an ultrasound system that (a) tracks a medical instrument in an anatomical region using (i) an ultrasound sensor mounted on the medical instrument and (ii) an ultrasound scan line pattern that spans an area of the anatomical region including the medical instrument, and (b) displays the ultrasound scan line pattern as a scanned image showing a position of the medical instrument by rendering an ROI on the scanned image, a method comprises: automatically and selectively (320) performing a ROI refinement by positioning a displayed portion of the scanned image such that the ROI is located in a preferred specific region of the displayed portion of the scanned image, wherein the displayed portion of the scanned image is a selectively magnifiable area within the area of the anatomical region including the medical instrument and spanned by the ultrasound scan line pattern.

It also should be understood that embodiments of the present invention will be described in terms of medical instruments; however, the teachings herein are much broader and are applicable to any ultrasound instruments. In some embodiments, the present principles are employed in tracking or analyzing complex biological or mechanical systems. In particular, the present principles are applicable to internal tracking procedures of biological systems and procedures in all areas of the body such as the lungs, gastro-intestinal tract, excretory organs, blood vessels, etc. The elements depicted in the figures, may be implemented in various combinations of hardware and software and provide functions which may be combined in a single element or multiple elements.

The functions of the various elements shown in the figures, can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (e.g., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray™ and DVD.

Reference in the specification to "one embodiment" or "an embodiment" of the present principles, as well as other variations thereof, means that a particular feature, structure, characteristic, and so forth described in connection with the embodiment is included in at least one embodiment of the present principles. Thus, the appearances of the phrase "in one embodiment" or "in an embodiment", as well any other variations, appearing in various places throughout the specification are not necessarily all referring to the same embodiment.

In interpreting the appended claims, it should be understood that:
(a) the word "comprising" does not exclude the presence of other elements or acts than those listed in a given claim;
(b) the word "a" or "an" preceding an element does not exclude the presence of a plurality of such elements;
(c) any reference signs in the claims do not limit their scope;
(d) several "means" may be represented by the same item or hardware or software implemented structure or function; and
(e) no specific sequence of acts is intended to be required unless specifically indicated.

It is to be appreciated that the use of any of the following "/", "and/or", and "at least one of', for example, in the cases of "A/B", "A and/or B" and "at least one of A and B", is intended to encompass the selection of the first listed option (A) only, or the selection of the second listed option (B) only, or the selection of both options (A and B). As a further example, in the cases of "A, B, and/or C" and "at least one of A, B, and C", such phrasing is intended to encompass the selection of the first listed option (A) only, or the selection of the second listed option (B) only, or the selection of the third listed option (C) only, or the selection of the first and the second listed options (A and B) only, or the selection of the first and third listed options (A and C) only, or the selection of the second and third listed options (B and C) only, or the selection of all three options (A and B and C). This may be extended, as readily apparent by one of ordinary skill in this and related arts, for as many items listed.

It will also be understood that when an element such as a layer, region or material is referred to as being "on" or "over" another element, it can be directly on the other element or intervening elements may also be present. In contrast, when an element is referred to as being "directly on" or "directly over" another element, there are no intervening elements present. It will also be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present.

Having described preferred embodiments for ultrasound object zoom tracking (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the embodiments disclosed herein as outlined by the appended claims. Having thus described the details and particularity required by the patent laws, what is claimed and desired protected by Letters Patent is set forth in the appended claims.

## Claims

1. An ultrasound device for imaging an anatomical region, comprising:
an ultrasound probe (204) having a medical instrument (202) in communication therewith, and an ultrasound sensor (279) mounted on the medical instrument; and
a hardware processor (214) configured to render a Region Of Interest (ROI) relative to a tip of the medical instrument on an ultrasound image displayed on a display device, and automatically and selectively perform a ROI refinement by positioning a displayed portion of the scanned image such that the ROI is located in a preferred specific region of the displayed portion of the scanned image, wherein the displayed portion of the scanned image is a selectively magnifiable area within an area of the anatomical region including the medical instrument and spanned by the ultrasound scan line pattern.

2. The system of claim 1, wherein the preferred specific region of the scanned image is a corner of the scanned image, and the processor (i) determines a trajectory of the medical instrument in the anatomical region and (ii) chooses the corner, from among corners of the scan line pattern, in accordance with the trajectory such that a subsequent movement of the medical instrument will advance the medical instrument toward the preferred specific region of the scanned image.

3. The system of claim 1, wherein the hardware processor (214) sets a magnification level of the displayed portion of the scanned image in accordance with a location of the medical instrument.

4. The system of claim 1, wherein the hardware processor (214) sets a magnification level of the displayed portion of the scanned image in accordance with a strength of a tracking signal of the medical instrument.

5. The system of claim 1, wherein a position of the ROI is fixed at a point on a display, and the scanned image is automatically panned as the medical instrument is moved in the area of the anatomical region.

6. The system of claim 1, wherein the hardware processor (214) alerts a user that the tip of the medical instrument is approaching a scan view edge limit by emphasizing an edge portion of the ROI corresponding to a trajectory direction of the medical instrument.

7. The system of claim 1, wherein the hardware processor (214) maintains a last pan position responsive to a medical instrument tracking signal, indicative of tracking success, falling below a threshold Signal To Noise Ratio (SNR).

8. The system of claim 6, wherein the hardware processor (214) updates the last pan position to a current pan position responsive to the medical instrument tracking signal rising to meet or exceed the SNR after having precedingly fallen below the threshold SNR.

9. The system of claim 1, wherein the hardware processor (214) automatically zooms in along a trajectory of the medical instrument when a medical instrument tracking signal, indicative of tracking success, exceeds a threshold Signal To Noise Ratio (SNR), and automatically zooms out when the medical instrument tracking signal meets or falls below the threshold SNR.

10. The system of claim 1, wherein the hardware processor (214) automatically zooms in along a trajectory of the medical instrument when a medical instrument tracking signal, indicative of tracking success, exceeds a first threshold Signal To Noise Ratio (SNR), and automatically zooms out when the medical instrument tracking signal meets or falls below a second threshold SNR, the first threshold being different than the second threshold for hysteresis-based zooming.

11. The system of claim 1, wherein the hardware processor (214) performs a position lock refinement that maintains the ROI at a center of the displayed portion of the scanned image.

12. The system of claim 11, wherein the position lock refinement permits boundary movement to the center of the displayed portion of the scanned image while truncating the displayed portion of the scanned image to prompt a user to move the medical instrument in to a new area to untruncate the displayed portion of the scanned image.

13. The system of claim 1, wherein the ROI refinement comprises automatically adjusting a zoom magnification and a zoom panning to position the medical instrument in the preferred specific location.

14. An ultrasound system for imaging an anatomical region, comprising:
an ultrasound probe (204) having a medical instrument (202) in communication therewith, and an ultrasound sensor (279) mounted on the medical instrument;
a display (218) configured to display an ultrasound scan line pattern, that spans an area of the anatomical region including the medical instrument, as a scanned image showing a position of the medical instrument by rendering an ROI on the scanned image; and
a hardware processor (214) configured to automatically and selectively perform a ROI refinement by positioning a displayed portion of the scanned image such that the ROI is located in a preferred specific region of the displayed portion of the scanned image, wherein the displayed portion of the scanned image is a selectively magnifiable area within an area of the anatomical region including the medical instrument and spanned by the ultrasound scan line pattern.

15. The ultrasound system of claim 14, wherein the preferred specific region of the scanned image is a corner of the scanned image, and the processor further runs the program code to (i) determine a trajectory of the medical instrument in the anatomical region and (ii) choose the corner, from among corners of the scan line pattern, in accordance with the trajectory such that a subsequent movement of the medical instrument will advance the medical instrument toward the preferred specific region of the scanned image.
